Europäisches Patentamt

European Patent Office

Office européen des brevets

(19)

(11) EP 0 623 001 B1

(12) EUROPEAN PATENT SPECIFICATION

(45) Date of publication and mention
of the grant of the patent:
**14.01.1998 Bulletin 1998/03**

(21) Application number: **92921185.2**

(22) Date of filing: **25.09.1992**

(51) Int Cl.[6]: **G01N 33/497**, A61B 5/00

(86) International application number:
**PCT/US92/08196**

(87) International publication number:
**WO 94/07407 (14.04.1994 Gazette 1994/09)**

(54) **BLOOD ALCOHOL MONITOR**

VORRICHTUNG ZUR ÜBERWACHUNG DES BLUTALKOHOLGEHALTES

DISPOSITIF DE SURVEILLANCE DU TAUX D'ALCOOLEMIE

(84) Designated Contracting States:
**DE GB SE**

(43) Date of publication of application:
**09.11.1994 Bulletin 1994/45**

(73) Proprietor: **Alcohol Monitoring Systems LLC
Denver, CO 80202 (US)**

(72) Inventors:
• **PHILLIPS, Mary, F.
Denver, CO 80228 (US)**
• **HAWTHORNE, Jeffrey, S.
Aurora, CO 80017 (US)**

(74) Representative: **Bibby, William Mark et al
Mathisen, Macara & co.
The Coach House
6-8 Swakeleys Road
Ickenham Uxbridge UB10 8BZ (GB)**

(56) References cited:
WO-A-89/03530          US-A- 3 649 199
US-A- 4 539 994          US-A- 4 996 161

**Description**

Specification

This invention relates to continuous monitoring of human blood alcohol levels and more particularly relates to a non-invasive blood alcohol level monitoring device. Even more particularly, the invention relates to a portable device, affixed to the subject, for monitoring blood alcohol, without requiring subject participation, by determining the alcohol levels expelled through a subject's skin.

Background and Field of the Invention

Devices for testing the blood and breath alcohol content of a human subject have been used for many years. This type of testing is typically done using breath alcohol testing methods, for example, see U.S. Patent 3,904,251, entitled "Apparatus for Detecting or Measuring a Constituent of a Gas", issued February 24, 1976 to Jones et al. This system tests the alcohol content of a subject's breath by utilizing a breath analyzing sensor in a breath sampling system that includes a disposable tube. A subject exhales into the tube and an operator of the handheld instrument pushes a button releasing a spring loaded pump which draws a deep lung breath sample into the device. The breath sample is then analyzed by a sensor and the subsequent reading is interpreted by the operator.

More recently such breath alcohol testing instruments have been added to Home Arrest Systems. These systems have an encoded transmitter and a base receiver unit. The transmitter is attached to the subject, and the system determines if the subject is located within a short distance, usually under 250 feet, from the base receiver unit. These systems may attach a conventional breath alcohol testing device to the base unit.

Other prior art, for example, U.S. Patent 4,843,377 issued June 27, 1989 to Fuller et al, and U.S. Patent 4,916,435 issued April 10, 1990 to Fuller, consists of a one way video system which is attached to a conventional breath alcohol tester. This system transmits a picture of the test results, along with a picture of the subject taking the test, over conventional phone lines to a central monitoring station. An operator at the central station must view the video picture to help assure that the proper subject is taking the breath alcohol test. U.S. Patent 4,665,385 entitled "Hazardous Condition Monitoring System", issued May 12, 1987 to Henderson consists of a hazardous gas sensor and an ethanol sensor located near a microphone attached to a walkie talkie. The system transmits encoded information pertaining to hazardous gas levels and subject breath alcohol levels when the walkie talkie is used.

U.S. Patent 4,997,770 entitled "Method and-Means for Detecting Blood Alcohol in Humans by Testing Vapor Above the Eye", issued March 5, 1991 to Giles et al, measures the blood alcohol content of vapors emitted by the human eye. This device requires that a cup be placed over the subject's eye, and a separate analyzer measures the alcohol content of gases collected in the cup.

All of the prior art described above requires the full cooperation of the subject to complete a successful breath test. Except for the system of Henderson, none of the remote alcohol systems described above are portable. Also, except for Giles et al, all of the prior art described above measures breath alcohol content.

Alcohol monitoring systems are currently needed to periodically measure a subject's blood alcohol level at a remote location over an extended period of time, such as, when a subject is in an alcohol rehabilitation program, or is under court order to not consume alcohol. For this type of system, identification of the subject providing the breath sample is a very significant problem. For example, an unsupervised remote breath alcohol tester being used by the subject is ineffective if an operator is not available to identify that the correct subject is breathing into the instrument. Because of the requirement of subject identification, these systems typically require the active participation and cooperation of the subject. Sometimes the subjects are uncooperative and will go to great lengths to avoid the testing. This increases the time and effort necessary to perform the testing, which often results in less testing being performed.

Another problem is that once a subject has been tested, the subject knows they will not be tested again for some period of time, such as, when the subject is involved in a work release or outpatient alcohol rehabiliation program. This allows the subject to leave the location of the stationary remote alcohol breath tester unit and immediately consume alcohol at high levels just after the testing. The subject is unlikley to show a high level of alcohol content if tested at a much later time because the rate of dissipation of alcohol from the human body is quite high.

Another major problem of all of these systems is the lack of any effective means to determine if a positive reading is created by ethanol in the testing subject's breath or blood, or by tampering with the instrument, such as, by pouring alcohol over the sensor in an effort to discredit the instrument. A wide variety of common interferant gases will react with the sensors utilized in the systems in a manner very similar to ethanol, which may provide a false positive reading, and it is important to be able to screen out interferant gasses. For a subject in an alcohol treatment program, a false reading may have severe consequences.

There is a need in the art then for an apparatus and method to passively test the blood alcohol content of a human subject. There is further need for such a system to passively confirm the identity of the subject during such testing. Still another need in the art is for a system to perform such testing automatically without the need of an operator, and without the need for cooperation from the subject being tested. A further need in the art is for such a system to perform frequent testing at either predetermined times, or at random times, and to perform such testing at any arbitrary location. Another important need unanswered by the prior art is a means of determining if a reading indicating the subject has consumed alcohol is caused by the blood alcohol levels of the subject or by numerous interferant gases which react with sensors in a manner similar to alcohol. The present invention satisfies these and other needs.

Summary of the Invention

According to a first aspect of the invention there is provided a portable monitor for sensing blood alcohol levels in a human subject comprising:

alcohol measuring means for measuring a percentage of alcohol contained in a gas within a predetermined distance from the human subject to create a percentage measurement;

timer means for activating said alcohol measuring means at predetermined time intervals to cause said alcohol measuring means to create a plurality of percentage measurements at said predetermined time intervals;

storage means for storing said plurality of percentage measurements; and

securing means for securing said alcohol measuring means within a selected distance from the human subject's skin.

According to a second aspect of the invention there is provided a method of monitoring the percentage of blood alcohol content of a human subject, said method comprising:

(a) placing a gas contents sensor in proximity to the human subject's skin;

(b) activating said sensor at predetermined time intervals to create a plurality of percentage measurements of the blood alcohol content in a gas sample emitted from the skin when said gas contents sensor is disposed at a predetermined distance from the skin;

(c) storing an error indication if the human subject tampers with said measurements or an error occurs within said measurements;

(d) storing each measurement of alcohol expelled through the subject's skin and storing a measurement result; and

(e) transmitting each of said measurement results and each of said indications to a monitoring station.

According to a third aspect of the invention there is provided a device for use in a monitoring system for determining blood alcohol levels in a human subject, and for attachment to a limb of the subject comprising:

a housing having an internal cavity therein;

gas content sensing means disposed in said internal cavity for measuring a percentage of alcohol contained in a gas expelled from a skin portion of the subject, said sensing means including a sample port containing a hydrophobic membrane and a fuel cell and valve means for opening and closing said sample port;

infrared sensing means in said internal cavity for determining the distance between the skin portion and said gas contents sensing means;

securing means including an electrically conductive strap encircling the limb of the subject for securing said housing to the subject with said internal cavity in facing relation to the skin portion and said gas contents sensing means a predetermined distance from the skin portion; and

programmable means including a timer for selectively opening said sample port and activating said fuel cell at predetermined time intervals, said programmable means including tamper detection means for detecting when said device is moved beyond a predetermined distance from the human subject.

It is an aspect of the present invention to perform testing which indicates the blood alcohol content of a human subject and to a novel and improved method and means for remote monitoring of a subject and in such a way as to avoid false readings.

It is another aspect of the invention to passively confirm the identity of the subject during such testing and monitoring.

Another aspect of the invention is to perform such testing and monitoring in a passive manner without requiring cooperation from the subject.

Yet another aspect is to perform such testing automatically without requiring an operator.

Still another aspect of the invention is to perform continuous testing and monitoring at predetermined times, typically every one-half hour, or to perform such testing at random times, regardless of the location of the subject and without the subject's knowledge that the test is being performed.

A further aspect of the invention is to perform such testing at any arbitrary location.

A still further aspect of this invention is to provide a means of determining whether output from the ethanol sensor is caused by the subject's blood alcohol levels or an interferant gas reacting with the ethanol sensor.

A still further aspect of the invention is to provide a means of reprogramming the invention from a remote location.

The above and other aspects of the invention are accomplished in a small, portable system that is attached to the subject by a secure, monitored strap. The system provides for the continuous monitoring of a subject's blood alcohol level by measuring the level of ethanol that has been expelled through the subject's skin. The system determines the subject's blood alcohol level by measuring the amount of ethanol at a predetermined distance away from the subject's skin, which provides an indication of the relative amount of ethanol in the subject's blood. The system also monitors the skin temperature of the subject prior to performing the alcohol test, thus providing an indication that a barrier has not been placed between the device and the subject's skin and to further confirm that the device has not been removed from the subject. Furthermore, the device may use a distance measuring system to measure the distance of the device from the subject's skin, to further confirm that the system has not been removed from the subject or that a barrier has not been placed between the subject's skin and the unit.

Typically, the device can be attached to a subject's arm or leg to provide periodic monitoring of the subject's blood alcohol content. The device cannot be removed by the subject, therefore, once attached, the device provides passive confirmation of the identification of the subject. If the subject attempts to remove the device, the device will detect such removal and provide an error indication. Because the device is small and portable, it is attached directly to the subject wherever the subject is located so that the measurement can be performed at any location and at any time, 24-hours a day, seven days a week.

The system contains a timer which allows the device to perform periodic blood alcohol measurements, typically every 30 minutes. The device can also be set to perform measurements at random times. The device performs such testing automatically, and requires no human operator's assistance or subject assistance to perform the measurements. Furthermore, the subject is unaware when a measurement is being taken, and is also unaware of the amount of alcohol measured by the device.

Periodically, the device is connected to a base unit which automatically transmits the positive, stored readings taken, device tamper error indications, and system diagnostic error indications, and the total number of tests performed, to a remote monitoring, on site, or a data collection center. Typically this connection is made once daily; however, the device is capable of storing readings for a period over 30 days.

The above and other aspects, features and advantages of the invention will be better understood by reading the following more particular description of the invention, presented in conjunction with the following drawings, wherein:

Brief Description of the Drawings

Figure 1 is a block diagram of the system of the present invention;
Figure 2 is a block diagram of the portable blood alcohol monitor of the system; Figure 3 shows a block diagram of the alcohol sensor of Figure 2;
Figure 4 is a block diagram of the temperator sensor of Figure 2;
Figures 5 through 9 show a flowchart of the software contained in the invention;
Figures 10 and 11 show a flowchart of the interferant detection process;
Figure 12 is a perspective view of a preferred form of bracelet in accordance with the present invention;
Figure 13 is a fragmentary view schematically illustrating the sensing chamber forming a part of the bracelet illustrated in Figure 12;
Figure 14 is a perspective view in detail of the valve control member for the sampling chamber contained in the bracelet; and
Figure 15 is a view partially in section of the sampling chamber in accordance with the present invention.

Detailed Description of Preferred Embodiment

The following description is of the best presently contemplated mode of carrying out the present invention. This description is not to be taken in a limiting sense but is made merely for the purpose of describing the general principles of the invention. The scope of the invention should be determined by referencing the appended claims.

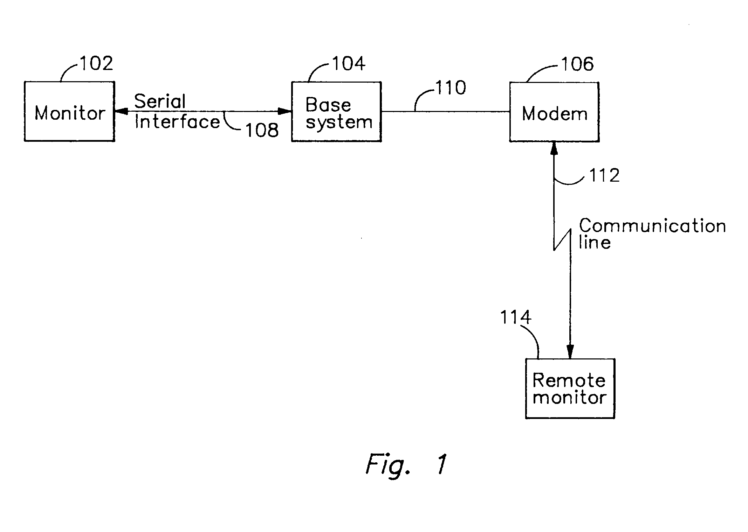

Figure 1 is a block diagram of the system of the present invention. Referring now to Figure 1, a portable blood alcohol monitor 102 is attached to the subject being monitored. Periodically, typically once per day, the subject or other person connects a serial interface 108 between the monitor 102 and a base system 104. When this connection is made, the monitor 102 transfers all of the readings, tamper, error indicators, and other data, taker since the last connection into the base system 104 where they are stored. The base system 104 transmits these readings at predetermined intervals over a modem connection 110 to a modem 106 which sends the readings over a communications line 112 to a remote monitor 114. Typically, the communications line 112 is a telephone system. The portable blood alcohol monitor 102 analyzes the readings taken to determine whether the subject's blood alcohol content has exceeded a predetermined level. If the alcohol content of the subject's blood has exceeded a predetermined level, a record is stored for transmission to the remote monitoring station 114 which can notify a person in authority that a high measurement has been taken.

The base system 104 can be a device such as a personal computer on site or at a data collection center, or it can be a device specifically designed to capture readings from the monitor 102 and transfer such readings to the remote monitoring station 114. In the preferred embodiment, the base system 104 is simply a store and forward device, and does not perform analysis of the readings taken by the monitor 102. In other embodiments of the invention, however, the base system 104 could be designed to perform such analysis and connect to the remote monitoring station 114 only if the subject's blood alcohol content exceeded a predetermined level or an error condition occurs. That is, while in the preferred embodiment the base system 104 simply stores the readings and transfers them to the remote monitoring station 114, the base system can monitor the readings and contact the remote monitoring station 114 when the readings exceeded predetermined levels or if there are any indications of a system malfunction, tampering, or a low battery condition.

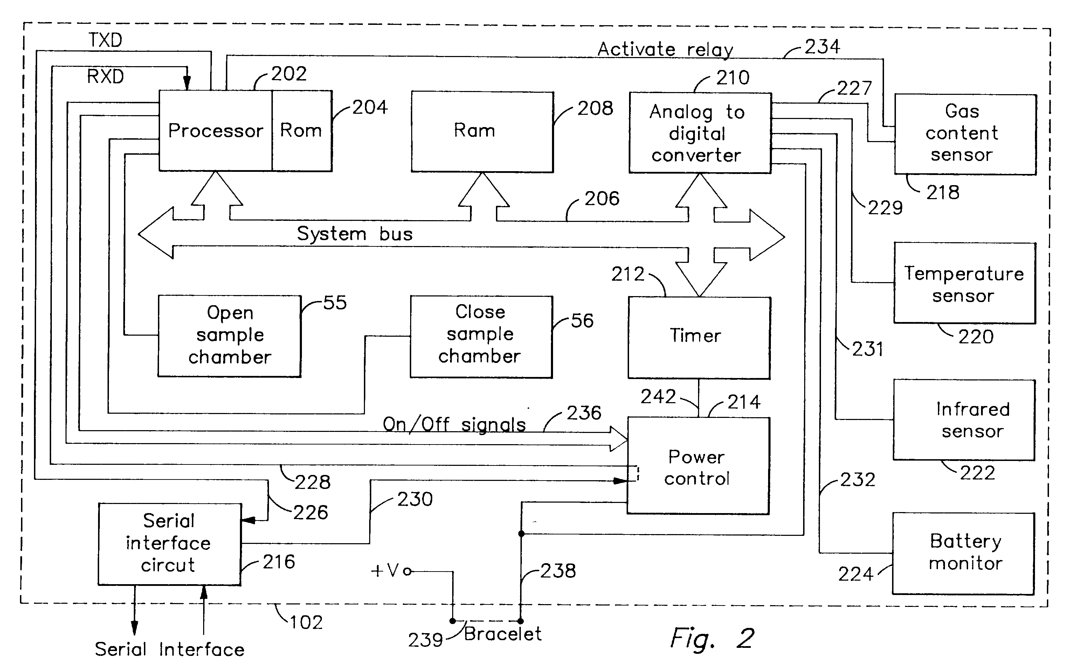

Figure 2 is a block diagram of the portable blood alcohol monitor 102 shown in Figure 1. Referring now to Figure 2, the monitor 102 contains a processor 202 which also contains read only memory, also called ROM, 204. The read only memory 204 is typically erasable, programmable read only memory, also called EPROM, which is built into the integrated circuit package that contains the processor 202. The software contained in this memory is a very basic program called a "boot strap" program. The boot strap program checks the validity of the main operating program contained in the random access memory (RAM) 208, establishes communication with a host computer system, not shown, or the base system 104 through the serial interface 108, and loads information, including the main operating program, into the monitor 102 through the serial interface 216.

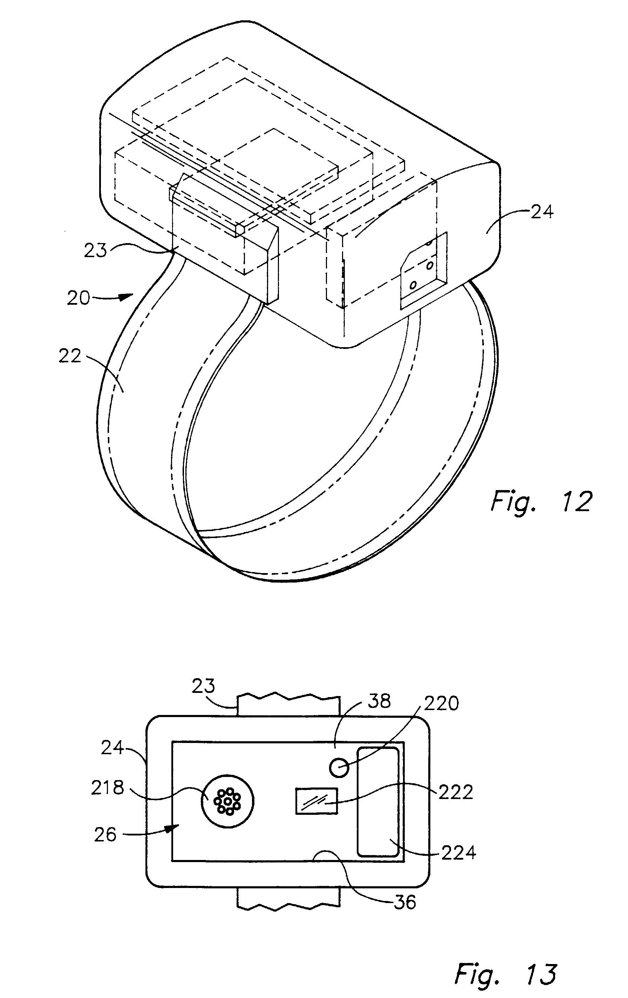

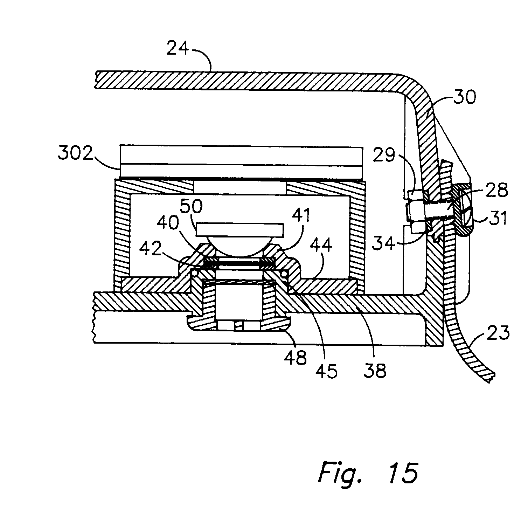

A preferred form of bracelet 20 is illustrated in Figures 12 to 15 and comprises a strap 22 having opposite ends 23 securely attached to an outer generally rectangular casing 24 for a sensing chamber 26 which is formed in the bottom of the casing 24. The strap 22 may be suitably composed of a 14.17g per 9.29dm$^2$ (one-half oz. per square foot) copper strip laminated on both sides over 0.01270cm (.005") Kapton (Trade Mark) material. This conductive layer is cut into widths of 2.22cm (7/8") and further laminated between a 2.54cm (1") wide layer of 0.01778cm (.007") Mylar (Trade Mark) on each side of the copper. The bracelet is permanently attached at opposite ends to the casing 24 by conductive fasteners 28, as shown in Figure 15, each fastener extending through the end 23 of the bracelet and through wall 30 of the casing 24 and firmly attached by a nut 29. Preferably, one end 23 of the bracelet is provided with an open slot to receive the fastener 28 so that the bracelet can be comfortably fitted to the subject's arm by sliding the slot on the bracelet relative to the fastener 28 and then tightening the fastener in position to firmly attach the bracelet to the casing 24. A star washer 32 is interposed between the enlarged end of the fastener and the end 23 of the bracelet, the washer penetrates through the Mylar laminate on the bracelet to make electrical contact with the copper sheeting contained within the strap 22 thereby establishing the conductive circuit 239. A watertight seal is effected by placing a silicone washer 34 between the nut 29 and inside of the casing wall 30, and the fastener 28 is covered on the outside by a small plastic tape 31. The fasteners 28 make electrical contact with conductor 238 within the casing 24 which is connected via conductive circuit to the main battery as illustrated in Figure 2. If the subject breaks the conductive circuit 239 while attempting to remove the monitor or bracelet 20, the signal over conductor 238 will become inactive and will cause the power control block 214 to supply power to the processor 202 which will immediately log the tamper error into RAM 208.

The processor 202 communicates with other elements of the monitor 102 over a system bus 206. A random access memory 208, which is used to hold the main operating program and the data measurements taken by the monitor 102, is connected to the system bus 206. In operation, the program from the ROM 204 is used to start the monitor and then transfer control to the main operating program in the RAM 208 after verifying that the main operating program is correct, or after loading a new copy of the main operating program through the serial interface 108. Although operating the main program from the RAM 208 provides higher performance, the main program could also be operated from the ROM 204.

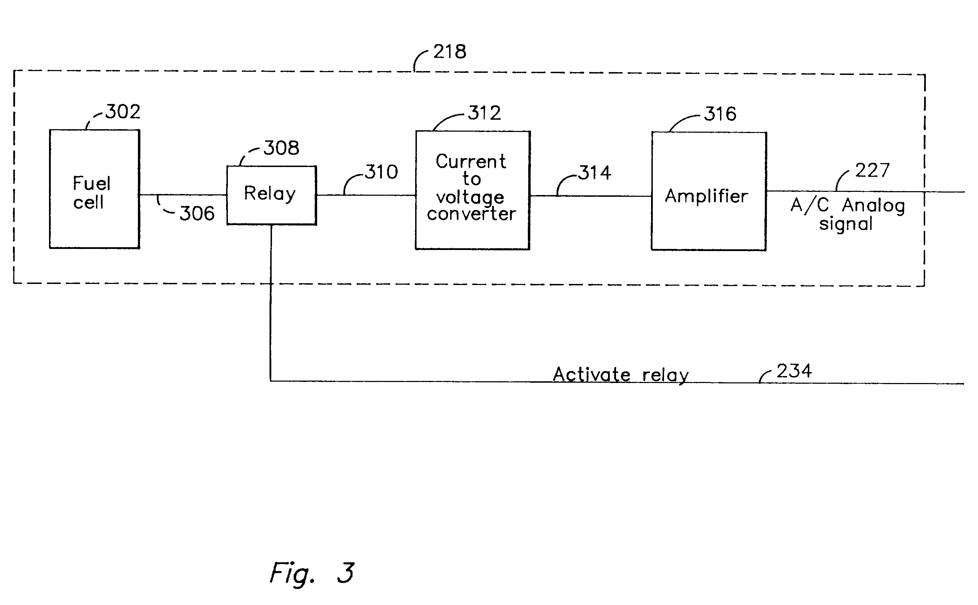

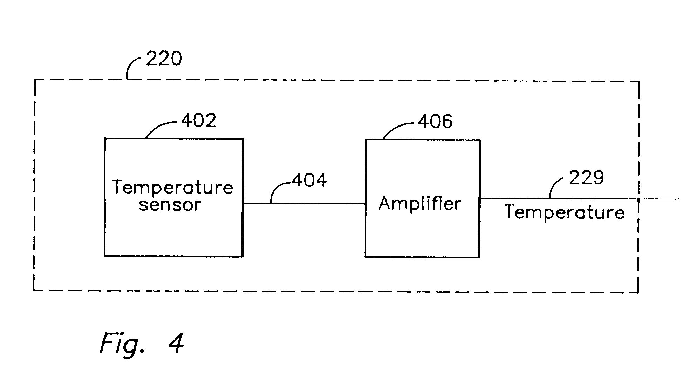

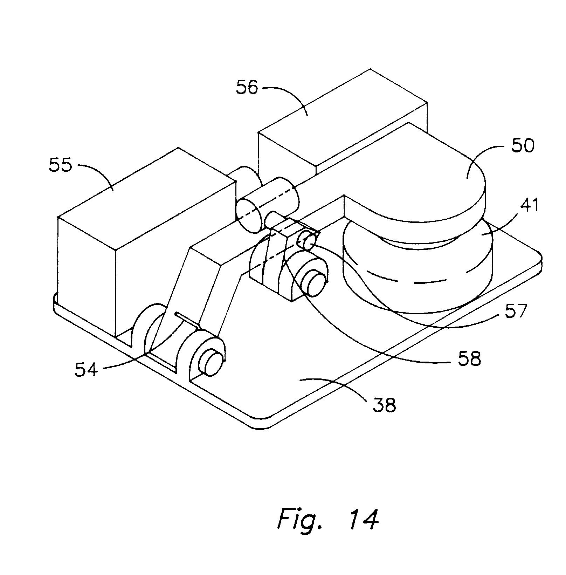

Also attached to the system bus 206 is the analog to digital converter 210 which receives signals via line 238 from the bracelet circuit 239. In the preferred form shown in Figure 13, the sensing chamber 26 is defined by an internal cavity 36 within the case 24 and which has a mounting plate 38 in its end wall spaced from the entrance to the cavity for a gas content sensor 218, a temperature sensor 220, an infrared sensor 222 and a battery monitor 224. As shown in more detail in Figures 14 and 15, the mounting plate 38 also supports a valve assembly including a membrane 40 disposed at one end of a sample port 41 and retained in position by a silicone washer 42. The outer wall of the port 41 receives a generally cup-shaped retainer portion 45 on the plate 38, the portion 45 having a central opening aligned with the port 41 and receives a perforated access screw 48 which is threaded into the open end of the portion 45. This is offset or recessed into the cup-shaped area 44 to retain the membrane 40 and washers 42 in position. One end of the port 41 opposite to the membrane 40 is normally closed by valve element 50 at the free end of a seal arm 52 which is pivotally mounted on the rear surface of the mounting plate 38 and is normally urged to a closed position by a spring element 54. A pair of solenoids 55 and 56 are disposed in spaced relation to one another to alternately control opening and closing of the seal arm 52 through a drive pin 57 and lift lever 58. When the solenoid 55 is activated through the processor 202, it will rotate the lever 58 upwardly to cause the drive pin 57 to engage the seal arm 52 and lift the valve element 50 away from the sample port 41. Conversely, when the solenoid 56 is activated, it will positively rotate the lever 58 downwardly to move the drive pin 57 away from the arm 52 so that the valve 50 is free to return to the closed position under the urging of the spring 54. When the bracelet is positioned on the wrist or ankle, preferably the inner end of the access screw 48 for the sample port is positioned a distance of 1 to 5 mm. away from the skin in order to be within an acceptable range for measurement. The analog to digital converter 210 converts data in analog form created by the gas content sensor 218, temperature sensor 220, infrared sensor 222, and battery monitor 224 into digital information which is input to the processor 202 over the system bus 206. After reading the information from the converter 210, the processor 202 stores the readings into the RAM 208. A block diagram of the gas content sensor 218 is shown in Figure 3, and a block diagram of the temperature sensor 220 is shown in Figure 4. The infrared sensor 222 is a specialized position sensitive detector integrated circuit, which is available as part number P2826, from Hamatsu Corp., Bridgewater, NJ, U.S.A. The battery monitor 224 has a voltage divider resistor network, not shown, connected to the main system battery, and the voltage divider outputs a signal which exceeds a logical "1" voltage when the battery is supplying a working voltage to the circuit, and outputs a logical "0" signal when the battery voltage has dropped below a usable working voltage.

As further shown in Figure 2, a timer circuit 212 is also attached to the system bus 206. The processor 202 sends data to the timer 212 to cause it to time the intervals between alcohol samples. After the processor has sent information to the timer to cause it to delay the amount of time to the next reading, the processor sends an "off" signal over "on/off signals" line 236 to a power control circuit 214 which causes the power control circuit 214 to remove power from the electronics of the system except for the timer 212 and the RAM 208. The RAM 208 has a separate backup battery which is always connected to the RAM 208.

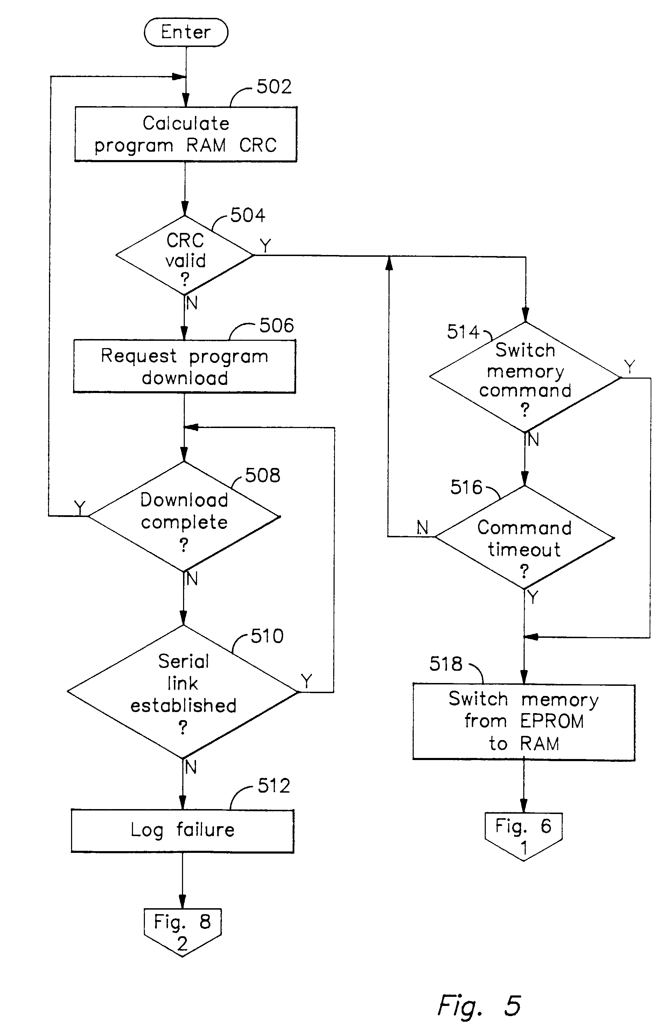

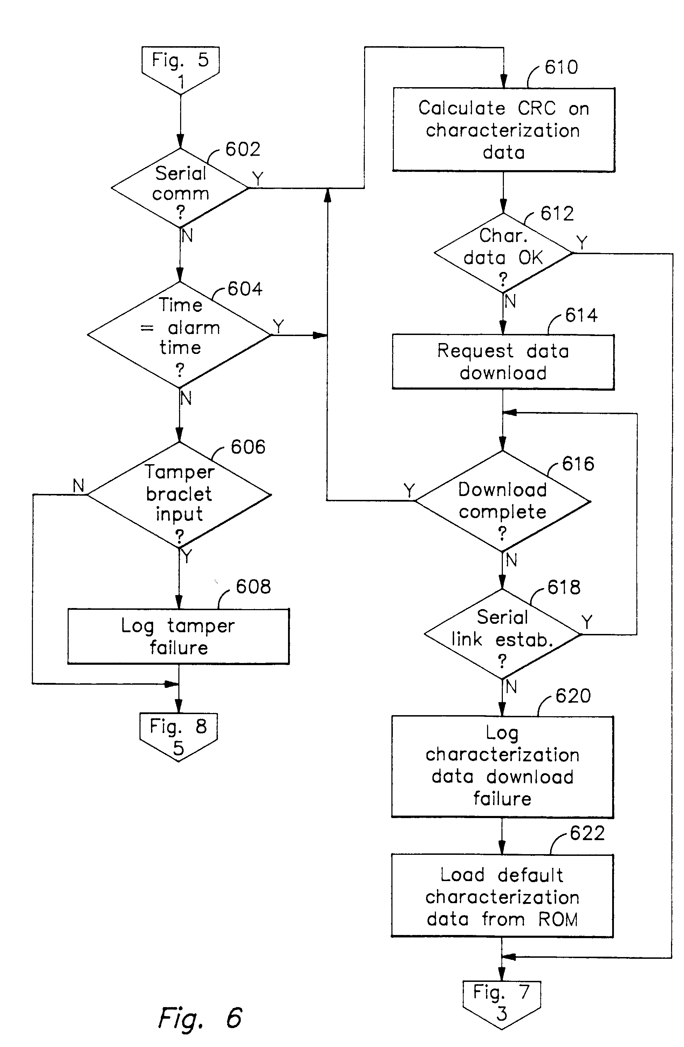

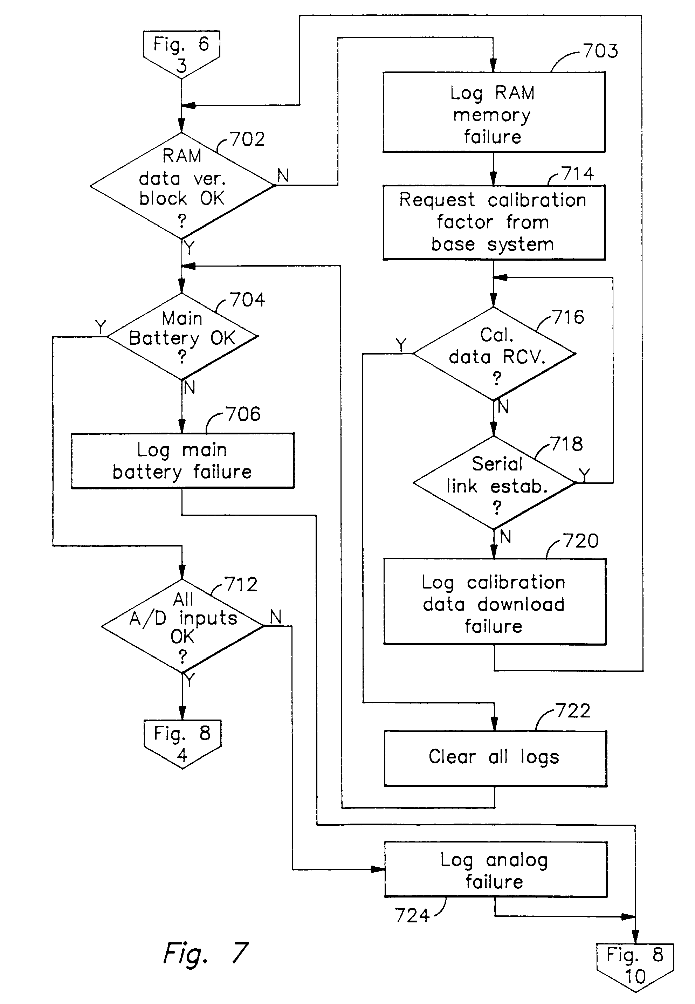

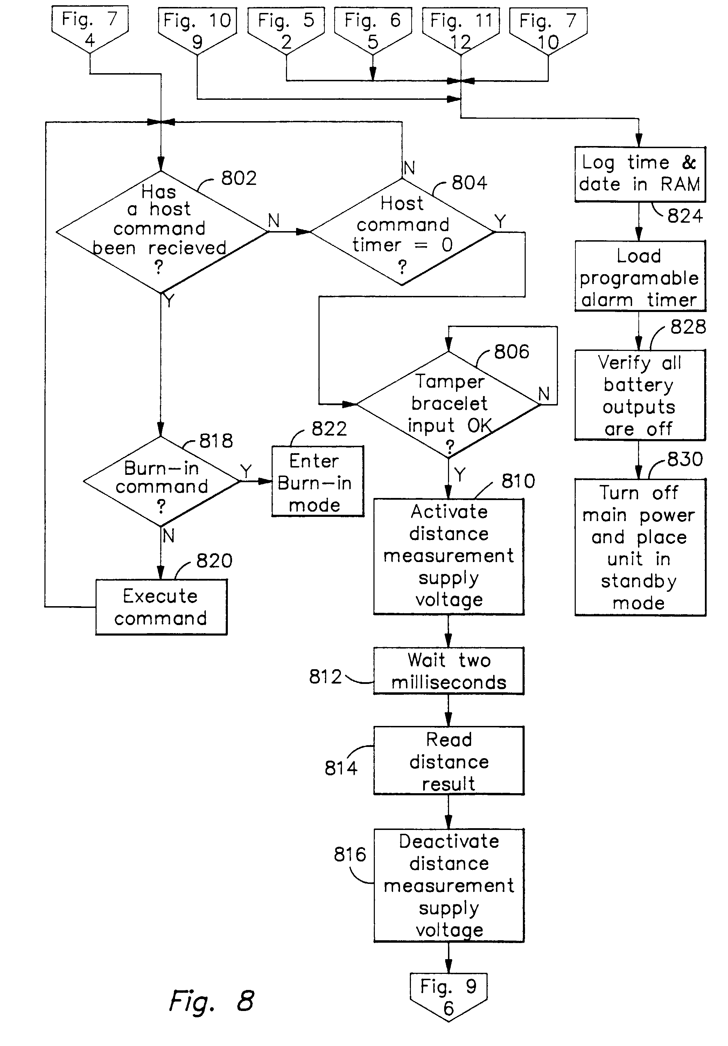

After the time has expired, the timer 212 outputs a signal 242 to the power control circuit 214 which reactivates power to the processor 202 and resets the processor 202 to start processing for the next measurement. The process of controlling the timer 212, and the power control 214, as well as the process for performing an alcohol content reading, will be described below with respect to Figures 5 through 10.

The monitor 102 also contains a serial interface circuit 216 which allows communications over a serial interface 108 to the base system 104 (Figure 1). The actual serial communications is controlled by the processor 202 by sending serial transmit data over line 226 through the serial interface circuit 216 and out the serial interface 108. The serial interface circuit 216 merely converts the voltage level of the transmit data signal 226 into a level that is suitable for the serial interface 108. When serial data is input over serial interface 108 through the serial interface circuit 216, the input data is sent over signal line 230 to the power control circuit 214. The signal loops through power control circuit 214 and becomes the receive data signal which is input over line 228 to the processor 202. As the receive data signal is passing through the power control circuit 214, it will cause the power control circuit 214 to supply power to the processor 202. Since the processor 202 is normally not active between taking alcohol readings, the processor 202 must be activated if the subject connects the monitor 102 to the base system 104 or personal computer. This activation is performed by receiving serial data over line 230.

Conductor circuit 239 within the bracelet 20 is connected to the analog to digital converter 210 as well as to the main battery. If the subject breaks the conducting circuit 239 contained in the bracelet while trying to remove the monitor, the signal over line 238 will become inactive. This will cause the power control block 214 to supply power to the processor 202 which allows the processor 202 to immediately log the tamper error into RAM 208.

Figure 3 shows a block diagram of the gas content sensor 218 of Figure 2. Referring now to Figure 3, the gas content sensor 218 contains a fuel cell 302 which is a model 7410 electro-chemical fuel cell available from Draggerwerk, located at Lubeck, Germany. The fuel cell 302 contains an opening or sample port 41, as shown in Figure 15, to allow the air sample to enter the fuel cell. To insure that the sample port 41 contains the proper gases to be analyzed, a Zitex A-150 and Gelman Fiberglass hydrophobic membrane 40 is located in the sample port 41 of the fuel cell 302. This membrane repels liquids while allowing gasses to flow through the sensor. The output 306 of the fuel cell 302 is an electrical current which is proportional to the amount of alcohol present in the air within the sample port 41. The sample port 41 is placed at a predetermined distance, typically 1 to 5 millimeters, above the subject's skin. The sensor measures the amount of alcohol being emitted through the subject's skin thereby providing an indication of the subject's blood alcohol content. The output 306 is connected to a relay 308 which is activated by an ACTIVATE RELAY signal 234 output by the processor 202 (Figure 2). As will be described below, the ACTIVATE RELAY signal causes a normally closed relay 308 to be opened to remove a short from the fuel cell output terminals. This allows the output 306 to flow through to line 310 which is input to a current to voltage converter circuit 312. The output 314 of the current to voltage converter 312 is amplified by an amplifier 316 to become the alcohol content analog signal 227 which is connected to the analog to digital converter 210 (Figure 2).

The relay 308 keeps the output of the fuel cell 302 shorted when the fuel cell is not being used to take a reading. When a reading is to be taken, the activate relay signal 234 causes the relay 308 to un-short the signal 306 which causes the fuel cell 302 to start to provide voltage output by the sensor reacting to any alcohol present within the sample port 41. The output 226 is then monitored by the processor 202 for approximately 30 seconds, to determine if the fuel cell has sensed alcohol present within the sample port. This process will be more fully described below with respect to Figures 5 through 11.

Figure 4 shows a block diagram of the temperature sensor circuit 220 of Figure 2. Referring now to Figure 4, the temperature sensor circuit 220 contains a precision fahrenheit temperature sensor integrated circuit 402. This device is a model LM34 precision fahrenheit temperature sensor available from Digi-Key, located at Thief River Falls, MN, U.S.A. The temperature sensor 402 provides a voltage output which increases linearly with an increase in temperature. The output 404 of the temperature sensor 402 is connected to an amplifier 406 which amplifies the signal to a level which can easily be converted by the analog digital converter circuit 210 (Figure 2). The output 229 of the amplifier 406 is connected directly to the analog to digital converter circuit 210.

Figure 5 shows a flowchart of the operation of the boot strap program which is contained in the ROM 204 (Figure 2). Figures 6 through 11 show a flowchart of the software of the main operating program of the present invention. This software is loaded through the serial interface 108 from the base system 104 (Figure 1) or from a host computer system (not shown), and stored in the RAM 208 (Figure 2).

The software starts execution whenever the processor 202 is reset. This reset can occur from three different sources. When the timer set by the previous measurement process expires, it causes the power control circuit 214 to supply power to the processor 202 and at the same time to reset the processor 202 to start execution of the software; or, if serial data is received over the serial interface 108, this data passes through the power control circuit 214 which also causes the processor to be reset; and the bracelet monitoring input 238 can reset the processor when a change in the voltage level being conducted by the bracelet circuit 239 is detected, indicating a tamper error.

Referring now to Figures 5 through 11, when the processor is reset, the software of Figure 5 is entered. When the software first starts execution, block 502 calculates the cyclic redundancy information (CRC) of the program stored in RAM 208 and determines whether the result indicates that the program was changed since it was loaded through the serial interface 108 into the RAM 208. Block 504 determines whether the CRC indicates that the program is valid.

If the program is not valid, block 504 transfers to block 506 which requests a new program download through the serial interface 108. Block 508 determines whether the download is complete and, if not, control transfers to block 510 which determines whether the serial link has been established or is still established. If the serial link was not established or has dropped, block 510 transfers to block 512 which logs a program load failure and then transfers to Figure 8 entry point number two to set a new timer value. If the serial link is still established, block 510 transfers back to block 508 until the download is complete. When the download is complete, block 508 transfers back to block 502 which recalculates the cyclic redundancy check information to check the new program code just loaded.

If the CRC is valid, thus indicating that the program code is intact, block 504.transfers to block 514 which determines whether a switch memory command has been received over the serial interface. If a switch memory command has been received, block 514 transfers to block 518. If a command has not been received, block 514 transfers to block 516 which determines whether the time to receive a command has expired. If the time has not expired, block 516 transfers back to block 514 and this loop continues until either a command is received or the time to receive a command has expired. After a command has been received or the time has expired, control transfers to block 518 which switches the program memory from the ROM 204 to the RAM 208 in order to execute the main operating system software. Control then transfers to Figure 6 entry point one, and to block 602.

Block 602 determines whether the serial communication is active and, if it is not, block 602 transfers to block 604 which determines whether the timer expired, therefore, whether the software should take another alcohol measurement. If serial communications is not active and the timer has not expired, control transfers to block 606 which determines whether the tamper bracelet input is inactive. If the tamper bracelet input is inactive, control goes to block 608 which logs a tamper failure into the RAM 208. After logging the failure or if the tamper bracelet input was active, control transfers to Figure 8 entry five to send a new timer value into the timer.

If the serial communications is active or the time had expired, control transfers to block 610 which calculates cyclic redundancy check information on characterization data stored within the RAM 208. The characterization data is data that was created by characterizing the fuel cell 302 (Figure 3) and is used to adjust the readings obtained from the fuel cell to obtain a more accurate percentage of alcohol content.

Block 612 then determines whether the characterization data is intact, by determining whether the cyclic redundancy check information indicates an error. If the characterization data is not intact, that is, it has been modified in some manner, block 612 transfers to block 614 which requests a download of characterization data from the serial interface. Block 616 then determines if the download is complete and, if not, transfers to block 618 which determines whether the serial link is still established. If the serial link is still established, block 618 transfers back to block 616 until the new characterization data has been downloaded and then control transfers back to block 610 to recalculate the cyclic redundancy check information. If the serial link has not been established or communication is broken during the transmission, control goes to block 620 which logs a data download failure into the RAM 208. Block 622 then loads default characterization data from the ROM 204 to use until new data can be downloaded. After loading default characterization data, or if the characterization data is intact and has not been modified, control transfers to Figure 7 entry point three, and block 702.

Block 702 determines whether the RAM data verification block is intact. The RAM data verification block is data stored in the RAM 208 that is used to determine whether the contents of the data within the RAM 208 have been modified. If the RAM data verification block is not valid, indicating that the data has been modified, block 702 transfers to block 703 which logs a RAM memory failure. Control then goes to block 714 which requests calibration data from the base system 104. Block 716 then determines whether the calibration data has been received and, if not, control goes to block 718 to determine whether the serial link has been established or is still established. If the link is still established, control transfers back to block 716 to loop until all the calibration data has been received. If the serial link is not established, control goes to block 702 which logs a calibration data download failure before transferring back to block 720. After the calibration data is received, block 716 transfers to block 722 which clears all log information within the RAM 208 and then transfers to block 704.

After clearing all log information or if the RAM data verification block is valid, control transfers to block 704 which samples the battery monitor 224 (Figure 2), through the analog to digital converter (Figure 2), to determine whether the main battery is still supplying sufficient power. If the battery is not supplying sufficient power, control goes to block 706 which logs a main battery failure before transferring to Figure 8 entry point 10 to turn off main power and place the system into standby mode.

If the main battery is supplying an acceptable voltage, block 704 transfers to block 712 which determines whether all other analog to digital inputs are valid and, if not, block 712 transfers to block 724 which logs an analog failure before transferring to Figure 8 entry point 10 to shut down the system. If all analog inputs are valid, block 712 transfers to Figure 8 block 802.

Block 802 determines whether a host command has been received from the base system 104 (Figure 1) and, if not, transfers control to block 804 which determines whether the time to receive a host command has expired. If the time has not expired, block 804 loops back to block 802 until either a host command is received or the wait time to receive a command has expired. If a host command is received, block 802 transfers to block 818 which determines whether the command received is a "burn-in" command. The burn-in command is used in initial manufacturing and testing of the unit, and if a burn-in command is received, block 818 transfers to block 822 which enters a test burn-in mode. The test burn-in mode puts the monitor unit into a slave configuration waiting to receive commands from the host computer system. The monitor can then be characterized. Characterization is the process of collecting the unit's sensor output voltage readings and storing these readings in RAM 208. The voltages are obtained under strictly controlled temperature conditions in an environmental chamber. A set of known alcohol standards is sampled by the monitor while in the chamber, and the resultant voltages are stored in RAM 208. The monitor remains in burn-in mode until the power is cycled or the monitor is reset.

If the command received is not a burn-in command, block 818 transfers to block 820 which performs the command before returning to block 802. The host commands performed by block 802 are primarily commands related to diagnostics, such as, diagnostic test, entering a slave mode for transferring programs back and forth between the monitor and the base system, auto-calibration diagnostics, transferring programs or transferring data. One of the host commands, however, is used to download the sample information which has been collected since the last download of this information to the host. That is, as each measurement is taken, a log is kept of the measurement and this log information is downloaded to the base system for eventual transfer to the remote monitor system.

If a host command is not received before the timer expires, control transfers to block 806 which determines if the bracelet circuit 239 is in place. If the bracelet circuit 239 is not in place, control will continue looping back to block 806 until the bracelet is in place. If the bracelet circuit 239 is in place then control is transferred back to block 810 which activates the infrared sensor 222 (Figure 2) to take a distance measurement. This activation is accomplished by programming the power control circuit 214 to supply power to the infrared sensor 222. After activating the distance measurement sensor, block 812 waits two milliseconds and then block 814 reads the distance value from the infrared sensor 222 via line 231, Figure 2. Block 816 then deactivates power to the distance measurement sensor to conserve battery power.

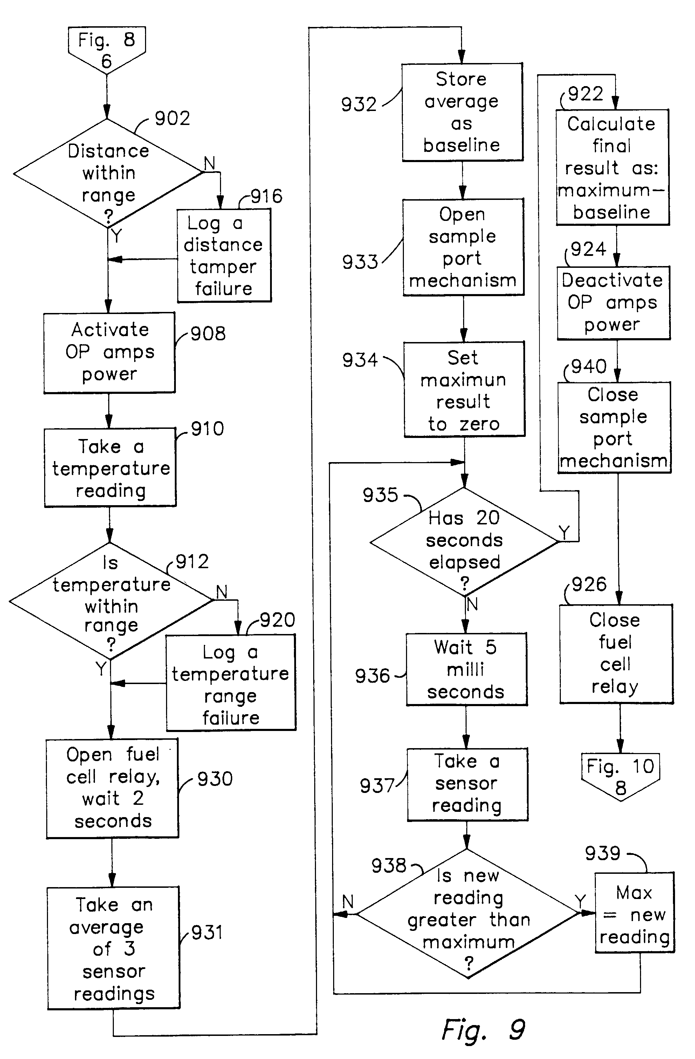

When control is transferred to entry point 6, Figure 9, block 902 determines whether the distance measured in block 814 is within an acceptable range for the device being attached to the subject. A typical distance is 1-5 millimeters. If the distance is not within an acceptable range, block 902 transfers to block 916 which logs a distance tamper failure into the RAM 208 and then transfers to block 908.

Block 908 then activates OP amps power to the temperature sensors and the gas content sensors. Block 910 reads the temperature from the temperature sensor 220 and block 912 determines whether the temperature is within an acceptable range for being located next to the subject. A typical range for this sensor is 94°F. to 98°F. If the temperature is not within an acceptable range, block 912 transfers to block 920 which logs a temperature failure into the RAM 208. After logging the failure or if the temperature is within range, control transfers to block 930 which opens the fuel cell relay 308 and then waits 2 seconds in order to let sensor voltage start to register. As discussed previously with respect to Figure 3, a relay 308 keeps the output of the fuel cell 302 shorted until a measurement is to be taken. Once the two seconds has expired, control transfers to block 931 which takes three independent readings of the fuel cell voltage from the gas content sensor 218 through the analog to digital converter 210, 5 milliseconds apart, and figures an average of the three readings. Block 932 is then used to store this average as a baseline reading. The baseline reading represents the output of the fuel cell with no alcohol present in the sample cavity. The sample port 41 is held in a sealed state by the arm 52 which must be opened as at block 933 by providing a voltage to the solenoid 55 which lifts the arm 52 enough to let the subject's ambient air enter the sample chamber. Block 934 then sets the maximum result to zero. Control then transfers to block 935 which checks for an elapsed total time of 20 seconds. If the total time elapsed is not greater than or equal to 20 seconds, then control transfers to block 936, which waits for 5 milliseconds. Control then transfers to block 937 which reads the fuel cell voltage of the gas content sensor 218 through the analog to digital converter 210. Block 938 then checks the resulting voltage of the reading against the maximum resulting voltage. If the result just read is greater than the maximum voltage, then control is transferred to block 939, where the result just read is stored as the maximum result. Control then transfers to block 935. If the resulting voltage of the reading is less than the maximum resulting voltage, control transfers immediately to block 935. The process described above continues until block 935 has reached a time of 20 seconds. Control then transfers to block 922 which calculates the total reading voltage as:

$$\text{Total Reading Voltage} = \text{Maximum} - \text{Baseline}$$

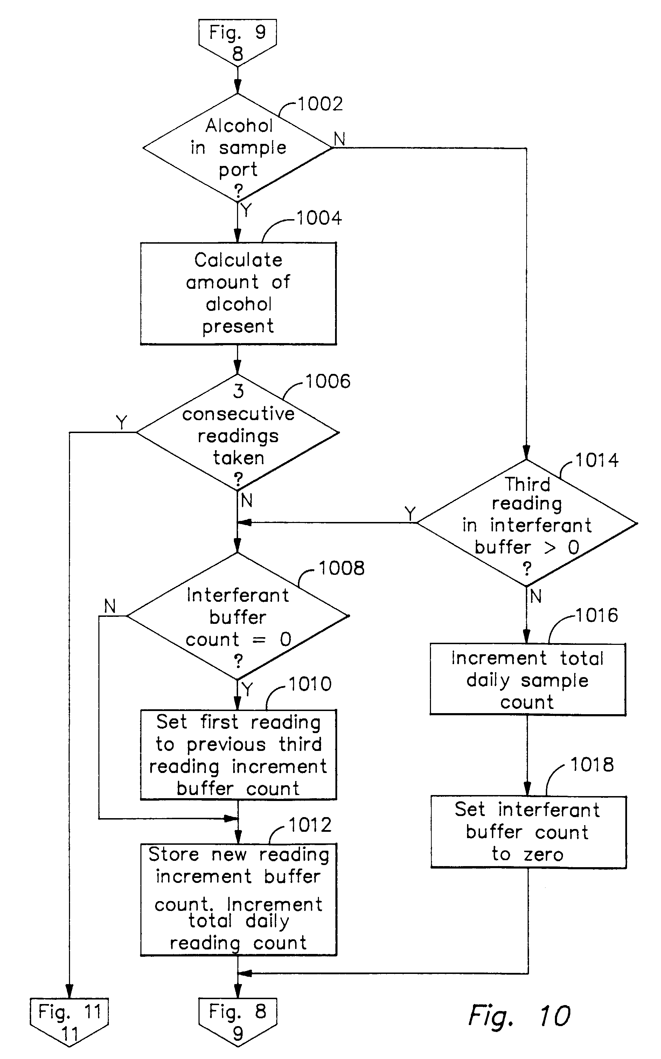

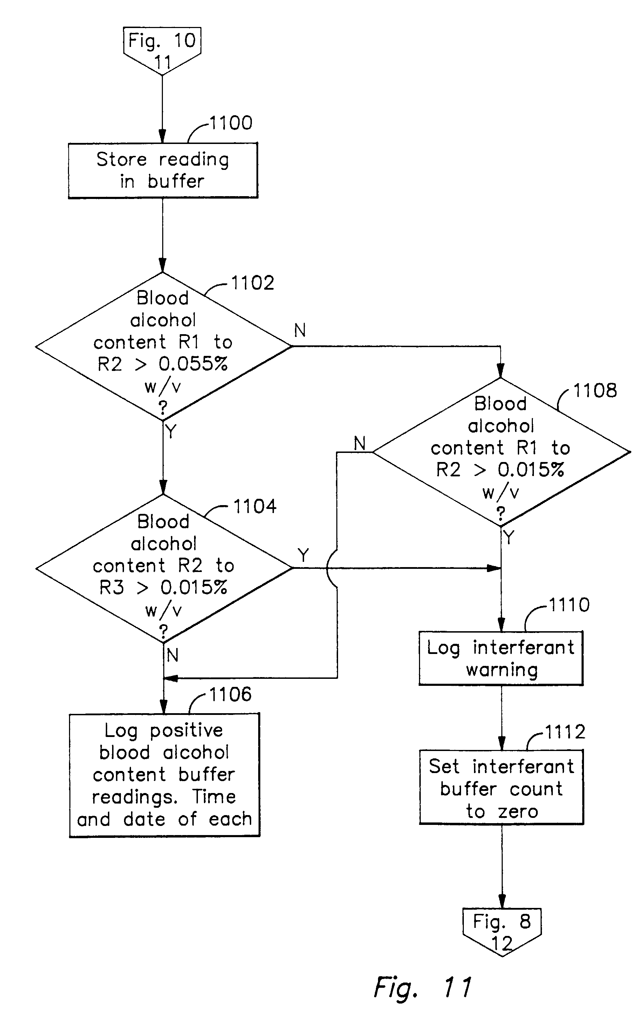

The total reading voltage is then stored. Control then transfers to block 924. Block 924 deactivates the OP amps power to turn the temperature sensor and gas content sensor operational amplifiers off. Block 940 will then close the arm 52 to again seal the sample port 41. This is done by providing a voltage to the solenoid 56 which will cause the arm 52 to close the sample port 41. Block 926 then deactivates the ACTIVATE RELAY signal over line 234 to close the fuel cell relay 308 and prevent it from taking additional measurements. Control then goes to Figure 10, block 1002.

Block 1002 determines if alcohol is present in the sample port at fuel cell 302. If alcohol is present, block 1004 then calculates the amount of alcohol present. The amount of alcohol present is calculated by comparing a reading from the sensor 218 to the known readings in the characterization data to produce a baseline blood alcohol result. This baseline reading is then multiplied by the calibration factor which compensates for sensor drift from the time the characterization was performed. This result is further multiplied by a temperature coefficient to provide the final amount of blood alcohol present. Block 1006 then determines if three consecutive samples have been taken and, if not, transfers to block 1008. Block 1008 determines if the interferant buffer count is zero, indicating that this is the first sample taken. If the count is zero, block 1008 transfers to block 1110 which sets the first interferant buffer reading to the previous third buffer reading and increments the buffer count.

After setting up the interferant buffer, or if the count is not zero, control goes to block 1012 which stores the new sample data in the interferant buffer, and increments the count of readings as well as the daily reading count. Control then goes back to Figure 8.

If no alcohol is in the sample port 41, block 1014 then determines whether the third reading in the interferant buffer is greater than zero. If the buffer reading is greater than zero, block 1014 transfers to block 1008 to store the sample reading just taken.

If the buffer reading is not greater than zero, block 1014 goes to block 1016 which increments the total daily sample count. Then block 1018 sets the interferant buffer count to zero to test the sampling process before transferring to Figure 8.

If three sample readings have been taken, block 1006 transfers to Figure 11, block 1100 to evaluate the readings. Block 1100 stores the reading just taken, and block 1102 determines if the absorption rate in the blood alcohol content between the first and second readings is greater than 0.055% weight per volume (W/V). As described in "The Metabolism Rates of Alcohol in the Human Body" by Robert B. Shumate et al, Journal of Forensic Medicine, Volume 14, Number 3, July-September, 1967, if the absorption rate is greater than 0.055% W/V or if the burn-off rate is greater than 0.015% W/V in a 30 minute period, the readings are not representative of alcohol content within a human body and must be due to an external source.

If the absorption rate is greater than 0.055% W/V, block 1102 transfers to block 1104 which determines if the burn-off rate betwen readings two and three is greater than 0.015% W/V. If this is not true, block 1104 goes to block 1106 to log the readings, including the date and time of each reading.

If block 1102 found that the adsorption rate was not greater than 0.055% W/V, it transfers to block 1108 which determines if the burn-off rate between readings two and three is greater than 0.015% W/V. If not, block 1108 transfers to block 1106 and logs a positive reading.

If the burn-off rate is greater than 0.015% W/V, both blocks 1104 and 1108 transfer to block 1110 which logs an interferant warning into the RAM 208. Block 1212 then sets the interferant count back to zero before returning to Figure 8.

Block 826 then loads the timer 212 with the next "wake up" time for taking the next measurement. In a first embodiment of the invention, the time between measurements is set at 30 minutes. In another embodiment, however, the time between measurements can be set to a random time, to allow for random measurements. When random timing is used, the total number of measurements within a 24-hour period is still set to 48, the same number of measurements that is taken with a regular 30-minute cycle. This insures that the data area within the RAM 208 is not exceeded.

After programming the timer, block 828 verifies that all optional battery outputs have been turned off to insure that there is no excessive drain on the batteries, and then block 830 turns off main power to place the monitor device into standby until the timer 212 expires and restarts the cycle.

Having thus described a presently preferred embodiment of the present invention, it will now be appreciated that the aspects of the invention have been fully achieved, and it will be understood by those skilled in the art that many changes in construction and circuitry and widely differing embodiments and applications of the invention will suggest themselves without departing from the spirit and scope of the present invention. The disclosures and the description herein are intended to be illustrative, the invention being more preferably defined in scope by the following claims.

**Claims**

1. A portable monitor (102) for sensing blood alcohol levels in a human subject comprising:

   alcohol measuring means (218) for measuring a percentage of alcohol contained in a gas within a predetermined distance from the human subject to create a percentage measurement; timer means (212) for activating said alcohol measuring means (20) at predetermined time intervals to cause said alcohol measuring means to create a plurality of percentage measurements at said predetermined time intervals; storage means (208) for storing said plurality of percentage measurements; and securing means (22) for securing said alcohol measuring means within a selected distance from the human subject's skin.

2. A portable monitor according to claim 1, wherein detection means (222) is provided for detecting when said measuring means (218) is greater than the selected distance from the human subject, and communication means (112) is provided for communicating said stored percentage measurements and distance measurements to another monitor (114).

3. A portable monitor according to claim 2, wherein a data processing center (114) is provided at said other monitor (114) to receive said percentage measurements and distance measurements from said communication means (112), and said other monitor (114) includes means (234) for activating said alcohol measuring means.

4. A portable monitor according to claim 1, wherein said securing means (22) includes:

   a bracelet (20) releasably attachable to a limb of the human subject; electrical conductor means (238) within said securing means; means (239) for causing an electrical current to be conducted through said electrical conductor means; and means (214) for detecting an absence of electrical current in said electrical conductor means.

5. A portable monitor according to claim 1, wherein there is provided:

   temperature measurement means (220) for measuring the human subject's skin temperature at a predetermined distance from the point where said alcohol measuring means is attached to the human subject; means (222) for detecting that said human subject's skin temperature is outside a predetermined range of temperatures; and means (104) for storing and transmitting to another monitor an indication of exceeding said range of temperatures.

6. A portable monitor according to claim 1, wherein said alcohol measuring means further comprises means (114) for determining that an interferant gas caused said predetermined range to be exceeded and further wherein said communication means (112) comprises means for sending an interferant gas indicator when said alcohol measuring means (218) determines that an interferant gas caused said predetermined range to be exceeded.

7. A method of monitoring the percentage of blood alcohol content of a human subject, said method comprising:

   (a) placing a gas contents sensor (218) in proximity to the human subject's skin; (b) activating said sensor (218) at predetermined time intervals to create a plurality of percentage measurements of the blood alcohol content in a gas sample emitted from the skin when said gas contents sensor is disposed at a predetermined distance from the skin;

(c) storing an error indication if the human subject tampers with said measurements or an error occurs within said measurements;

(d) storing each measurement of alcohol expelled through the subject's skin and storing a measurement result; and

(e) transmitting each of said measurement results and each of said indications to a monitoring station.

8. The method of claim 7 wherein step (c) further comprises the steps of:

(a) determining if a temperature measurement of the human subject's skin is within a predetermined range of temperatures; and

(b) if a change in said percentage of alcohol exceeds a predetermined increase during a predetermined time period, of storing an interferant indication transmitting said interferant indication to said monitor station.

9. A device for use in a monitoring system for determining blood alcohol levels in a human subject, and for attachment to a limb of the subject comprising:

a housing (24) having an internal cavity (36) therein;

gas content sensing means (218) disposed in said internal cavity for measuring a percentage of alcohol contained in a gas expelled from a skin portion of the subject, said sensing means (218) including a sample port (41) containing a hydrophobic membrane (40) and a fuel cell (302) and valve means (50) for opening and closing said sample port (41);

infrared sensing means in said internal cavity (36) for determining the distance between the skin portion and said gas contents sensing means (218) ;

securing means (20) including an electrically conductive strap (22) encircling the limb of the subject for securing said housing (24) to the subject with said internal cavity (36) in facing relation to the skin portion and said gas contents sensing means (218) a predetermined distance from the skin portion; and

programmable means (202) including a timer (212) for selectively opening said sample port (41) and activating said fuel cell (302) at predetermined time intervals, said programmable means (202) including tamper detection means (222) for detecting when said device is moved beyond a predetermined distance from the human subject.

10. A device according to claim 9, additionally comprising

ing means for directing an electrical current through said conductive strap (22), means for detecting an absence or change of electrical current in said conductive strap (22), and means for transmitting any such absence or change of electrical current to a remote location.

**Patentansprüche**

1. Tragbare Überwachungseinrichtung (102), die Blutalkoholspiegel bei einem menschlichen Wesen erfaßt, und die umfaßt:

eine Alkoholmeßeinrichtung (218), die einen Prozentsatz von in einem Gas innerhalb eines vorgegebenen Abstandes zu dem menschlichen Wesen enthaltenen Alkohol mißt, um eine Prozentsatzmessung zu erzeugen;

eine Zeitgebereinrichtung (212), die die Alkoholmeßeinrichtung (20) in vorgegebenen Zeitintervallen aktiviert, um zu bewirken, daß die Alkoholmeßeinrichtung eine Vielzahl von Prozentsatzmessungen in den vorgegebenen Zeitintervallen erzeugt;

eine Speichereinrichtung (208), die die Vielzahl von Prozentsatzmessungen speichert; und

eine Befestigungseinrichtung (22), mit der die Alkoholmeßeinrichtung innerhalb eines vorgegebenen Abstandes zu der Haut des menschlichen Wesens befestigt wird.

2. Tragbare Überwachungseinrichtung nach Anspruch 1, wobei eine Erfassungseinrichtung (222) vorhanden ist, die erfaßt, wenn die Meßeinrichtung (218) größer ist als der ausgewählte Abstand zu dem menschlichen Wesen, und eine Übertragungseinrichtung (112) vorhanden ist, die die gespeicherten Prozentsatzmessungen und Entfernungsmessungen zu einer anderen Überwachungseinrichtung (114) überträgt.

3. Tragbare Überwachungseinrichtung nach Anspruch 2, wobei ein Datenverarbeitungszentrum (114) in der anderen Überwachungseinrichtung (114) vorhanden ist, das die Prozentsatzmessungen und die Entfernungsmessungen von der Übertragungseinrichtung (112) empfängt, und die andere Überwachungseinrichtung (114) eine Einrichtung (234) enthält, die die Alkoholmeßeinrichtung aktiviert.

4. Tragbare Überwachungseinrichtung nach Anspruch 1, wobei die Befestigungseinrichtung (22) enthält:

ein Armband (20), das lösbar an einer Extremität des menschlichen Wesens angebracht werden kann;

eine elektrische Leitereinrichtung (238) in der Befestigungseinrichtung;

ein Einrichtung (239), die bewirkt, daß ein elektrischer Strom durch die elektrische Leitereinrichtung geleitet wird; und

eine Einrichtung (214), die ein Nichtvorhandensein elektrischen Stroms in der elektrischen Leitereinrichtung erfaßt.

5. Tragbare Überwachungseinrichtung nach Anspruch 1, wobei vorhanden sind:

eine Temperaturmeßeinrichtung (220), die die Hauttemperatur des menschlichen Wesens in einem vorgegebenen Abstand zu dem Punkt mißt, an dem die Alkoholmeßeinrichtung an dem menschlichen Wesen angebracht ist;

eine Einrichtung (222), die erfaßt, daß die Haupttemperatur des menschlichen Wesens außerhalb eines vorgegebenen Bereiches von Temperaturen liegt; und

eine Einrichtung (104), die eine Anzeige des Überschreitens des Bereiches von Temperaturen speichert und zu einer anderen Überwachungseinrichtung übermittelt.

6. Tragbare Überwachungseinrichtung nach Anspruch 1, wobei die Alkoholmeßeinrichtung des weiteren eine Einrichtung (114) umfaßt, die bestimmt, daß ein Störgas bewirkt hat, daß der vorgegebene Bereich überschritten wurde, und wobei des weiteren die Übertragungseinrichtung (112) eine Einrichtung umfaßt, die eine Störgasanzeige sendet, wenn die Alkoholmeßeinrichtung (218) bestimmt, daß ein Störgas bewirkt hat, daß der vorgegebene Bereich überschritten wurde.

7. Verfahren zum Überwachen des Prozentsatzes des Alkoholgehaltes im Blut eines menschlichen Wesens, wobei das Verfahren umfaßt:

(a) Anbringen eines Gasgehaltsensors (218) in der Nähe der Haut des menschlichen Wesens;

(b) Aktivieren des Sensors in vorgegebenen Zeitintervallen, um eine Vielzahl von Prozentsatzmessungen des Blutalkoholgehaltes in einer Gasprobe zu erzeugen, die von der Haut abgegeben wird, wenn der Gasgehaltsensor in einem vorgegebenen Abstand zu der Haut angeordnet ist;

(c) Speichern einer Fehleranzeige, wenn das menschliche Wesen in die Messungen eingreift oder ein Fehler innerhalb der Messungen auftritt;

(d) Speichern jeder Messung von Alkohol, der durch die Haut des Wesens ausgestoßen wird und Speichern eines Meßergebnisses; und

(e) Übermitteln jedes der Meßergebnisse und jeder der Anzeigen zu einer Überwachungsstation.

8. Verfahren nach Anspruch 7, wobei Schritt (c) des weiteren die folgenden Schritte umfaßt:

(a) Bestimmen, ob eine Messung der Hauttemperatur des menschlichen Wesens innerhalb eines vorgegebenen Bereiches von Temperaturen liegt; und

(b) wenn eine Veränderung des Prozentsatzes von Alkohol während eines vorgegebenen Zeitraums einen vorgegebenen Anstieg übersteigt, Speichern einer Störanzeige und Übermitteln der Störanzeige zu der Überwachungsstation.

9. Vorrichtung zum Einsatz in einem Überwachungssystem zum Bestimmen von Blutalkoholpegeln bei einem menschlichen Wesen und zum Anbringen an einer Extremität des Wesens, die umfaßt:

ein Gehäuse (24) mit einem Innenhohlraum (36);

eine Gasgehalterfassungseinrichtung (218), die in dem Innenhohlraum angeordnet ist, um einen Prozentsatz von in einem Gas, das von einem Hautabschnitt des Wesens ausgestoßen wird, enthaltenen Alkohol zu messen, wobei die Erfassungseinrichtung (218) eine Prüföffnung (41) enthält, die eine wasserabweisende Membran (40) sowie eine Brennstoffzelle (302) und eine Ventileinrichtung (50) zum Öffnen und Schließen der Prüföffnung (41) enthält;

eine Infraroterfassungseinrichtung in dem Innenhohlraum (36), die den Abstand zwischen dem Hautabschnitt und der Gasgehalterfassungseinrichtung (218) bestimmt;

eine Befestigungseinrichtung (20), die einen elektrisch leitenden Gurt (22) enthält, der die Extremität des Wesens umschließt, um das Gehäuse (24) an dem Wesen zu befestigen, wobei der Innenhohlraum (36) dem Hautabschnitt zugewandt ist und die Gasgehalterfassungseinrichtung (218) in einem vorgegebenen Abstand zu dem Hautabschnitt ist; und

eine programmierbare Einrichtung (202), die einen Zeitgeber (212) enthält, der die Prüföffnung (41) selektiv öffnet und die Brennstoffzelle (302) in vorgegebenen Zeitintervallen aktiviert, wobei die programmierbare Einrichtung (202) eine Einrichtung (222) zum Erfassen unbefugten Eingriffs enthält, die erfaßt, wenn die Vorrichtung über einen vorgegebenen Abstand zu dem menschlichen Wesen hinaus bewegt wird.

10. Vorrichtung nach Anspruch 9, die zusätzlich eine Einrichtung, die einen elektrischen Strom durch den leitenden Gurt (22) leitet, eine Einrichtung, die ein Nichtvorhandensein bzw. eine Veränderung elektrischen Stroms in dem leitenden Gurt (22) erfaßt, und eine Einrichtung umfaßt, die jedes derartige Nichtvorhandensein bzw. jede derartige Veränderung elektrischen Stroms zu einer entfernten Stelle übermittelt.

**Revendications**

1. Dispositif de surveillance portable (102) pour détecter des taux d'alcoolémie chez un sujet humain, comprenant :

un moyen de mesure du taux d'alcool (218) pour mesurer un pourcentage d'alcool contenu dans un gaz jusqu'à une mesure en pourcentage;
un moyen de minuterie (212) pour activer ledit moyen de mesure du taux d'alcool (20) à des intervalles de temps prédéterminés pour entraîner ledit moyen de mesure du taux d'alcool à créer une pluralité de mesures en pourcentage auxdits intervalles de temps prédéterminés;
un moyen de mémorisation (208) pour mémoriser ladite pluralité de mesures en pourcentage; et
un moyen de fixation (22) pour fixer ledit moyen de mesure du taux d'alcool jusqu'à une distance sélectionnée de la peau du sujet humain.

2. Dispositif de surveillance portable selon la revendication 1, dans lequel un moyen de détection (22) est prévu pour détecter quand ledit moyen de mesure (218) est éloigné de plus de la distance sélectionnée du sujet humain, et un moyen de communication (112) est prévu pour communiquer lesdites mesures en pourcentage mémorisées et lesdites mesures de distance à un autre dispositif de surveillance (114).

3. Dispositif de surveillance portable selon la revendication 2, dans lequel un centre de traitement des données (114) est prévu au niveau dudit autre dispositif de surveillance (114) pour recevoir lesdites mesures en pourcentage et lesdites mesures de distance depuis ledit moyen de communication (112), et ledit autre dispositif de surveillance (114) comporte un moyen (234) pour activer ledit moyen de mesure du taux d'alcool.

4. Dispositif de surveillance portable selon la revendication 1, dans lequel ledit moyen de fixation (22) comporte :

un bracelet (20) attachable de manière amovible à un membre du sujet humain;
un moyen conducteur électrique (238) à l'intérieur dudit moyen de fixation;
un moyen (239) pour provoquer l'entraînement d'un courant électrique à travers ledit moyen conducteur électrique ; et
un moyen (214) pour détecter une absence de courant électrique dans ledit moyen conducteur électrique.

5. Dispositif de surveillance selon la revendication 1, dans lequel il est prévu :

un moyen de mesure de la température (220) pour mesurer la température de la peau du sujet humain à une distance prédéterminée du point où ledit moyen de mesure du taux d'alcool est attaché au sujet humain;
un moyen (222) pour détecter que ladite température de la peau du sujet humain est en dehors d'une plage prédéterminée de températures; et
un moyen (104) pour mémoriser et transmettre à un autre dispositif de surveillance une indication d'un dépassement de ladite plage de températures.

6. Dispositif de surveillance portable selon la revendication 1, dans lequel ledit moyen de mesure du taux d'alcool comprend en outre un moyen (114) pour déterminer qu'un gaz de brouillage a provoqué le dépassement de ladite plage prédéterminée et en outre dans lequel ledit moyen de communication (112) comprend un moyen pour envoyer un indicateur de gaz de brouillage lorsque ledit moyen de mesure du taux d'alcool (218) détermine qu'un gaz de brouillage a provoqué le dépassement de ladite plage prédéterminée.

7. Procédé de surveillance du taux d'alcoolémie d'un sujet humain, ledit procédé comprenant les étapes consistant à :

(a) placer un capteur de teneur en gaz (218) à proximité de la peau d'un sujet humain;
(b) activer ledit capteur (218) à des intervalles de temps prédéterminés pour créer une pluralité de mesures en pourcentage du taux d'alcoolémie dans un échantillon gazeux émis par la peau lorsque ledit capteur de la teneur en gaz est disposé à une distance prédéterminée de la peau;
(c) mémoriser une indication d'erreur si le sujet humain tente de modifier lesdites mesures ou si une erreur s'introduit dans lesdites mesures;
(d) mémoriser chaque mesure de l'alcool émis à travers la peau du sujet et mémoriser un résultat de mesure; et
(e) transmettre chacun desdits résultats de mesure et chacune desdites indications à un poste de surveillance.

8. Procédé selon la revendication 7, dans lequel l'étape (c) comprend en outre les étapes consistant à :

(a) déterminer si une mesure de la température de la peau du sujet humain est dans une plage de températures prédéterminée; et
(b) mémoriser une indication de brouillage en transmettant ladite indication de brouillage audit poste de surveillance si un changement dudit taux d'alcool dépasse une augmentation prédéterminée durant une période de temps prédéterminée.

9. Dispositif pour l'utilisation dans un système de surveillance pour déterminer des taux d'alcoolémie chez un sujet humain, et pour l'attacher à un membre du sujet, comprenant :

un boîtier (24) ayant une cavité interne (36);
un moyen de détection de la teneur en gaz (218) disposé dans ladite cavité interne pour mesurer un taux d'alcool contenu dans un gaz émis par une portion de peau du sujet, ledit moyen de détection (218) comportant un port d'échantillonnage (41) contenant une membrane hydrophobe (40) et une cellule électrochimique (302) et un moyen de soupape (50) pour ouvrir et fermer ledit port d'échantillonnage (41);
un moyen de détection infrarouge dans ladite cavité interne (36) pour déterminer la distance entre la portion de peau et ledit moyen de détection de la teneur en gaz (218) ;
un moyen de fixation (20) comportant une lanière (22) électriquement conductrice encerclant le membre du sujet pour fixer ledit boîtier (24) au sujet avec ladite cavité interne (36) en relation face à face avec la portion de peau et ledit moyen de détection de la teneur en gaz (218) à une distance prédéterminée de la portion de peau; et
un moyen programmable (202) comportant une minuterie (212) pour ouvrir de manière sélective ledit port d'échantillonnage (41) et activer ladite cellule électrochimique (302) à des intervalles de temps prédéterminés, ledit moyen programmable (202) comportant un moyen de détection des manipulations (222) pour détecter quand ledit dispositif est déplacé au-delà d'une distance prédéterminée du sujet humain.

10. Dispositif selon la revendication 9, comprenant en outre un moyen pour diriger un courant électrique à travers ladite lanière conductrice (22), un moyen pour détecter une absence ou un changement de courant électrique dans ladite lanière conductrice (22), et un moyen pour transmettre une telle absence ou un tel changement de courant électrique quelconque à un emplacement éloigné.

*Fig. 1*

EP 0 623 001 B1

Fig. 2

EP 0 623 001 B1

*Fig. 3*

220

402

406

Temperature
sensor

404

Amplifier

229

Temperature

Fig. 4

Fig. 5

Fig. 5
1

↓

602
Serial
comm
?
─Y→

N
↓

604
Time
= alarm
time
?
─Y→

N
↓

606
N← Tamper
braclet
input
?

Y
↓

608
Log tamper
failure

↓

Fig. 8
5

610
Calculate CRC on
characterization
data

↓

612
Char.
data OK
?
─Y→

N
↓

614
Request data
download

↓

616
Y← Download
complete
?

N
↓

618
Serial
link estab.
?
─Y→

N
↓

620
Log
characterization
data download
failure

↓

622
Load default
characterization
data from ROM

↓

Fig. 7
3

*Fig. 6*

*Fig. 7*

Fig. 7 / 4    Fig. 10 / 9    Fig. 5 / 2    Fig. 6 / 5    Fig. 11 / 12    Fig. 7 / 10

**802** — Has a host command been recieved ?

**804** — Host command timer = 0 ?

**824** — Log time & date in RAM

**828** — Load programable alarm timer

Verify all battery outputs are off

**830** — Turn off main power and place unit in standby mode

**806** — Tamper bracelet input OK ?

**818** — Burn-in command ?

**822** — Enter Burn-in mode

**820** — Execute command

**810** — Activate distance measurement supply voltage

**812** — Wait two milliseconds

**814** — Read distance result

**816** — Deactivate distance measurement supply voltage

Fig. 9 / 6

*Fig. 8*

Fig. 8
6

902 — Distance within range ? — N → 916 — Log a distance tamper failure

Y ↓

908 — Activate OP amps power

910 — Take a temperature reading

912 — Is temperature within range ? — N → 920 — Log a temperature range failure

Y ↓

930 — Open fuel cell relay, wait 2 seconds

931 — Take an average of 3 sensor readings

932 — Store average as baseline

933 — Open sample port mechanism

934 — Set maximun result to zero

935 — Has 20 seconds elapsed ? — Y →

N ↓

936 — Wait 5 milli seconds

937 — Take a sensor reading

938 — Is new reading greater than maximum ? — Y → 939 — Max = new reading

N ←

922 — Calculate final result as: maximum—baseline

924 — Deactivate OP amps power

940 — Close sample port mechanism

926 — Close fuel cell relay

Fig. 10
8

*Fig. 9*

Fig. 9
8

1002
Alcohol
in sample
port
?

N

Y

1004
Calculate
amount of
alcohol
present

3
consecutive
readings
taken
?
1006

Y

N

1014
Third
reading
in interferant
buffer > 0
?

Y

N

1008
Interferant
buffer
count = 0
?

N

Y

1010
Set first reading
to previous third
reading increment
buffer count

1016
Increment total
daily sample
count

1012
Store new reading
increment buffer
count. Increment
total daily
reading count

1018
Set interferant
buffer count
to zero

Fig. 11
11

Fig. 8
9

*Fig. 10*

*Fig. 11*

Fig. 12

Fig. 13

Fig. 14

*Fig. 15*